# EUROPEAN PATENT APPLICATION

(11) **EP 4 151 730 A1**
(43) Date of publication of application: **22.03.2023**
(21) Application number: 21306274.8
(22) Date of filing: 15.09.2021
(51) Int. Cl.: C12N 15/113, A61K 31/713

(54) **TREATMENT OF COVID-19 WITH REVERSE MICELLE SYSTEM COMPRISING UNMODIFIED OLIGONUCLEOTIDES**

(71) Applicant: Medesis Pharma, 34670 Baillargues (FR); Centre national de la recherche scientifique, 75016 Paris (FR); UNIVERSITE DE MONTPELLIER, 34090 Montpellier (FR)
(72) Inventor: SEITZ, Hervé, GRABELS 34790 (FR); MOURI, Abdelkader, 34070 MONTPELLIER (FR); MAUREL, Jean-Claude, 34160 CASTRIES (FR)
(74) Representative: Cabinet Becker et Associés

(57) **Abstract**

The present invention relates to specific reverse micelle system of the invention which allows the administration and intracellular delivery of unmodified oligonucleotide, such as siRNA, targeting one or more genes of the SARS-CoV-2 virus. The reverse micelle system of the invention is thus particularly useful for the treatment of COVID-19.

## Description

### FIELD OF THE INVENTION

The present invention relates to specific reverse micelle system of the invention which allows the administration and intracellular delivery of unmodified oligonucleotide, such as siRNA, targeting one or more genes of the SARS-CoV-2 virus. The reverse micelle system of the invention is thus particularly useful for the treatment of the viral pathology linked to the SARS-CoV-2 virus.

### BACKGROUND OF THE INVENTION

Coronavirus disease 2019 (COVID-19) is an infectious disease caused by the severe acute respiratory syndrome coronavirus 2 (SARS-CoV-2). The disease has spread globally since 2019, resulting in the 2019-21 coronavirus pandemic. Common symptoms include fever, cough and shortness of breath. Muscle pain, sputum production and sore throat are less common symptoms. While the majority of cases result in mild symptoms, some progress to pneumonia and multi-organ failure. The deaths per number of diagnosed cases is estimated at between 1% and 5% but varies by age and other health conditions.

There is consequently a major public health emergency to treat CoVID_19 which is spreading worldwide very quickly.

RNAi (RNA interference) and antisense (AS) strategies consist in silencing the expression of a target gene by the use of nucleic acids which allow the degradation or the translational arrest of mRNA target. New antisense applications (exon skipping, alternative splicing correction), by masking the mutation responsible for an alternative splicing default, have permitted the synthesis of a functional protein. Aptamers are nucleic acids capable of interacting with a target protein and down regulating its synthesis. The discovery of all these nucleic acids, and more recently siRNA and miRNA, has opened wide perspectives in therapeutics for the treatment of diseases like genetic diseases, cancers, neurodegenerative diseases, infectious and inflammatory diseases or to block cell proliferation and diseases caused thereby.

However, these molecules are unstable in biological fluids, *in vitro* and *in vivo,* they display a poor intracellular penetration and low bioavailability. These critical drawbacks have limited their use in therapeutics. As a result, clinical applications of said nucleic acids have required chemical modifications with the aim of retaining their capacity to knockdown protein expression while increasing stability and cellular penetration. Research groups have also applied the nanotechnology approach to improve their delivery, to overcome most barriers that hampered the development of nucleic acids delivery-based therapies. To improve bioavailability, many researchers have also attempted to use alternative administration routes: ocular, skin, oral, intramuscular. Those attempts have not been totally satisfactory so far. For instance, some of these attempts, more specifically assays with nucleic acids in liposome carriers have stimulated immune response.

The object of the present invention is to overcome disadvantages of the prior art. There is an obvious need for a safe and efficient nucleic acids therapeutic strategy for the treatment of diseases related to SARS-CoV-2 virus (or for the treatment of COVID-19), and in particular for new tools that are able to achieve efficient gene expression modulation-based therapy in order to treat diseases related to SARS-CoV-2 virus.

### SUMMARY OF THE INVENTION

It is an object of the invention to provide a drug delivery system comprising an unmodified oligonucleotide targeting one or more genes of SARS-CoV-2, which can be for instance administered via buccal mucosa, giving rise to a satisfactory drug bioavailability in an active form.

The present invention relates to a delivery system for the *in vivo,* or *ex vivo* release of unmodified oligonucleotides targeting SARS-CoV-2 RNAs, by administration to the buccal or rectal mucosa of said delivery system, as well as the compositions and methods for preparing the delivery system.

More specifically, the delivery system is a reverse micelle system comprising at least one sterol, acylglycerol, phospholipid, an alcohol, and at least one unmodified oligonucleotide targeting one or more genes of SARS-CoV-2 virus.

Herein described are reverse micelle systems designed to reach this goal in a safe and controlled manner. The reverse micelle systems are able to be absorbed through mucosa and to vectorize unmodified oligonucleotides under a protected form to all cells of any tissue of the organism.

The invention also relates to a pharmaceutical composition comprising a reverse micelle system as defined herein and a pharmacologically acceptable support or carrier.

The present invention relates to the use of unmodified oligonucleotides targeting CoV_2019 RNAs, in particular siRNAs whose sequences have been designed to inhibit one or more genes expression of this virus, in the preparation of reverse micelle systems or pharmaceutical compositions comprising the same in the treatment of diseases related to SARS-CoV-2 (or in the treatment of COVID-19).

The aim of the present invention is to provide unmodified oligonucleotides targeting SARS-CoV-2 RNAs in a delivery system that allows to vectorize said oligonucleotides as to down regulate or knock down the expression of a target nucleic acid of SARS-CoV-2 virus, with high efficiency and limited off-target-mediated secondary effects.

Drug delivery technology according to the invention allows intracellular delivery to all tissues and organs using HDL lipoprotein (High Density Lipoprotein) or vHDL lipoprotein (Very-High-Density Lipoprotein) receptors.

More particularly, the present invention provides a reverse-micelle transport system for delivering unmodified oligonucleotides capable of modulation of gene expression or duplication of genes of SARS-CoV-2 virus. More specifically, reverse micelles according to the invention allow the incorporation thereof in HDL and vHDL lipoprotein in the buccal or rectal mucosa. Reverse micelles according to the invention are thus carried in a protected lymphatic transport form, then in the general blood circulation which finally allows an intracellular delivery of said oligonucleotides by the membrane receptors of HDL lipoproteins, of the SRB-1 type (Scavenger receptor class B type 1).

Advantageously, at no time can the subject's immune system detect the presence of the oligonucleotide, with absence of immune reaction when unmodified oligonucleotides are administered in the technology according to the invention.

The reverse micelles can be prepared according to a method described below using at least a sterol, an acylglycerol, a phospholipid, an alcohol, water, and at least one unmodified oligonucleotide targeting one or more genes of SARS-CoV-2 virus.

Said micelles are more particularly obtainable by the following method:
(a) Contacting (i) sterol, preferably sitosterol or cholesterol, (ii) acylglycerol, preferably diacylglycerol of fatty acids, (iii) phospholipid, preferably phosphatidylcholine, (iv) alcohol, (v) water, preferably purified water, and (vi) at least an unmodified oligonucleotide targeting one or more genes of SARS-CoV-2 virus,
(b) Stirring mixture obtained in step (a), at 40 °C or less, and for a time sufficient to obtain formation of reverse micelles, said stirring being preferably carried out mechanically.

The parameters of the mechanical stirring, for instance duration and speed, can be readily determined by anyone skilled in the art and depend on experimental conditions. In practice, these parameters are such that a micro-emulsion is obtained; the speed is determined so as to enable formation of a visually transparent formulation, and duration of the stirring is such that the stirring may be stopped a few minutes after obtaining the visually transparent formulation.

The present invention further relates to a pharmaceutical composition comprising reverse micelles of the invention and a pharmaceutically acceptable carrier.

### LEGENDS TO THE FIGURES

**Figure 1** shows siRNA duplexes of the invention.
**Figure 2** shows graphs of viability of VeroE6 cells transfected with various concentrations of siRNAs before SARS-CoV-2 infection.
**Figure 3** shows that transfection of 20 nM siRNA in VeroE6 cells significantly reduces viral replication (assessed by RT-qPCR on viral RNA).

### DETAILED DESCRIPTION OF THE INVENTION

The following description is of preferred embodiments by way of examples only and without limitation to the combination of features necessary for implementing the invention.

### Reverse micelles

The reverse micelle system according to the invention is characterized as a micro-emulsion comprising a dispersion of water-nanodroplets in oil. The dispersion is stabilised by two surfactants (acylglycerol, more preferably a diacylglycerol of fatty acids and a phospholipid, more preferably phosphatidylcholine) and a co-surfactant (alcohol) that are most likely at the water/oil interface. The reverse micelle phase can be defined as a system wherein water forms the internal phase and the hydrophobic tails of the lipids form the continuous phase. Reverse micelles containing oil(s), surfactant(s), co-surfactant(s), and an aqueous phase are also characterized as water-in-oil micro-emulsions.

Generally, the size of micelles according to the invention is very small, more particularly, it is less than 10 nm; more specifically it is less than 8 nm and more preferably less than 5 nm. The size may vary with the quantity of added water and phospholipid. The present invention relates more particularly to reverse micelles with an aqueous core of 3 to 5 nm, preferably from 3.5 to 5 nm, in particular from 3.7 to 4.5 nm.

The reverse micelles and the size of their aqueous core can be characterized by various methods, including:
- Small Angle X-Ray Scattering (SAXS)
- Neutrons Scattering
- Transmission Electron Microscopy (TEM)
- Dynamic Light Scattering (DLS)

The ratios of the lipidic constituents (including sterol, acylglycerol and phospholipid) in the reverse-micelle system according to the invention can vary. For instance, the weight ratio sterol/acylglycerol can range from 0.015 to 0.05, more particularly from 0.03 to 0.04. The weight ratio phospholipid/acylglycerol is from 0.06 to 0.25. For the calculation of these ratios, the weight of phospholipid corresponds to the total weight of the mixture of phospholipids, for instance the weight of lecithin, used in the formulation.

The compounds of the reverse-micelle system can be analysed by appropriate means. More specifically, sterols can be identified by gas chromatographic analysis and acylglycerol by high-performance liquid chromatography (HPLC), in particular with a light scattering detector, on a silica column (kromasil C18), in the presence of an eluent, e.g. isocratic acetonitrile. Gas chromatography can also be used to analyse diacylglycerols. Phospholipids can be analysed by high-performance liquid chromatography (HPLC), with a diol column with a light scattering detector.

Reverse micelles are dynamic systems. Brownian motion causes perpetual collisions of micelles, which lead to coalescence of micelles and exchange of the aqueous cores. Separation and regeneration of micelles occur and allow chemical reactions between different solutions. The exchange rate between micelles increases in particular with temperature, the length of hydrocarbon chains of the surfactant, and the water/surfactant ratio. Within the context of the invention and contrary to what is expected in nanotechnology, aqueous cores of micelles must have a specific size allowing one or more molecules of unmodified oligonucleotide, in particular nucleic acid capable of mediating RNA interference, to be stabilised in the prepared micelles. As mentioned above, the size of the aqueous core is around 4 nm, preferably from 3 to 5 nm, more preferably from 3.5 to 5 nm, in particular from 3.7 to 4.5 nm.

Without being bound to any theory, it seems that inclusion of a phospholipid in the reverse micelle system allows formation of micelles with greater diameter and volume, thus allowing vectorization of greater quantities of oligonucleotide.

In addition, it seems that, when applied to mucosa tissue, the reverse micelle system triggers formation of lipoproteins which after a lymphatic transport then in the blood circulation cross the cellular membrane and allow delivery of the oligonucleotide, in particular the nucleic acid capable of modulating gene expression of SARS-CoV-2 virus into the cells.

After the deposition of the micro emulsion on the buccal mucosa (or rectal mucosa) in the subject, the Brownian dynamics of the reverse micelles promotes intramucosal penetration into the intercellular spaces, and in contact with the apoproteins present physiologically in the mucosa, there takes place a structure in lipoproteins vHDL and HDL.

Oligonucleotides must be soluble in water, so as not to interfere with the water/oil interface of the reverse micelles according to the invention.

An amphiphilic molecule modifies the water solubility in the nano micelles, interferes with the interface and removes the fluidity of the permanent Brownian-like motions of the micelles which is necessary for their passage in the mucosa and their absorption through the structuration in lipoproteins.

The oligonucleotides described in the present invention are necessarily unmodified in order to be water-soluble.

Accordingly, the invention ensures absorption of the compounds to be delivered across mucosa, preferably across mouth, nasal and/or rectal mucosa, more preferably across mouth mucosa. Also, reverse micelles of the present invention provide an important bioavailability with low variability of absorption.

### Method for preparing reverse micelles

In a particular embodiment, the invention relates to a method for preparing reverse micelles as defined above (involving more specifically at least one unmodified oligonucleotide targeting one or more genes of SARS-CoV-2 virus, a sterol, an acylglycerol, a phospholipid, an alcohol, and water), wherein said method comprises the following steps :
(a) Contacting (i) sterol, (ii) acylglycerol, preferably diacylglycerol of fatty acids, (iii) phospholipid, preferably phosphatidylcholine, (iv) alcohol, (v) water, preferably purified water, and (vi) at least one unmodified oligonucleotide capable of targeting one or more genes of SARS-CoV-2 virus,
(b) Stirring mixture obtained in step (a), at 40 °C or less, and for a time sufficient to obtain formation of reverse micelles, said stirring being carried out mechanically.

The obtained and recovered reverse micelles are then particularly useful as a delivery system for unmodified oligonucleotides. Step (b) of the process is of particular importance since it allows reverse micelles to be obtained, said reverse micelles being then useful as a transport system to deliver unmodified oligonucleotides directly into the cytoplasm of all cells in all tissues and organs, through the cell membrane lipoprotein receptors.

In a particular embodiment, the unmodified oligonucleotide is first solubilised in water (preferably purified water) to form an aqueous phase. Said aqueous phase is then introduced into the oily phase (according to step(a)). The oily phase preferably comprises at least a sterol, an acylglycerol, a phospholipid and an alcohol.

The compounds involved in step (a) will be described in more details below.

Stirring of the mixture obtained by step (a) is carried out at a temperature less than or equal to 40°C, preferably ranging from 30 °C to 38 °C, more preferably from 30 °C to 35 °C, for a time sufficient to form of reverse micelles. The time sufficient can vary in particular upon the used stirring techniques, i.e., mechanical stirring. The time of mechanical stirring is more specifically the time needed to convert the initial mixture into a visually transparent reverse micelle solution.

One skilled in the art knows how to select excipients or components that may be used along with the composition according to the present invention in order to keep their beneficial properties. In particular, the presence of glycerol can, when introduced in large amount, prevent the formation of reverse micelles or break the reverse micelle system. More specifically, no more than 2.5%, and preferably no glycerol (percent expressed by weight of glycerol / weight of acylglycerol) is used for the preparation of the reverse micelles of the present invention.

Other compounds can be introduced in step (a). One can cite for instance colouring agents and/or flavouring substances.

In an advantageous manner, the compounds cited above or the commercially available mixtures containing them are the only ingredients introduced to prepare the micelle system and consequently the only ones present in the micelle system of the invention.

Physical parameters, in particular time- for instance comprised between 3 and 5 minutes, in one or several times-, are dependent on the used material, volumes of the mixture and viscosity thereof. One skilled in the art can readily define such parameters. Temperature of the mixture is less than 40°C. Such a temperature avoids degradation of the reactants. Temperature is preferably ranging from 30 °C to 38 °C, more preferably from 30 °C to 35 °C.

The usual materials use propellers whose fast movements generate turbulences and swirls allowing interpenetration of particles and formation of reverse micelles within the mixture.

Stirring speed is preferably ranging from 200 to 2 000 r/minute, more preferably from 300 to 700 r/minute. The implemented volumes, device, and stirring speed depend on and should be adapted with the reactants and amounts thereof.

As described above, temperature of the mixture must not exceed 40°C. Temperature is preferably ranging from 30 °C to 38 °C, more preferably from 30 °C to 35 °C.

### REVERSE MICELLES COMPOUNDS

### ACYLGLYCEROL

Acylglycerols, more particularly acylglycerols of fatty acids, useful for the preparation of the reverse-micelle system according to the invention can be isolated from the majority of animals and more preferably plants.

Acylglycerols can be mono- and/or diacylglycerols. In a particular embodiment, mono- or diacylglycerols preferentially used in the present invention present the following formula (I): 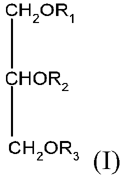 in which:
- R₁ is an acyl residue of a linear or branched, saturated or unsaturated fatty acid having between 14 and 24 carbon atoms, a hydrogen atom, or a mono-, di- or tri-galactose or glucose;
- R₂ is an acyl residue of a linear or branched, saturated or unsaturated fatty acid having between 2 and 18 carbon atoms;
- R₃ is an acyl residue of a linear or branched, saturated or unsaturated fatty acid having between 14 and 24 carbon atoms, or a hydrogen atom.

According to a particular embodiment, R₁ or R₃, preferably only one of R₁ and R₃, in particular only R₁, represents an acyl residue of oleic acid (C18: 1[cis]-9), including in particular glycerol monooleate.

According to a particular aspect, R₂ has one unsaturated bond (e.g; ethylenic bond) and has advantageously 18 carbon atoms, preferably R₂ is an oleic acid residue (oleoyl group), one of its positional isomers with respect to the double bond (cis-6,7,9,11 and 13) or one of its iso-branched isomers.

According to another particular aspect, R₁ represents an oleoyl group.

According to another particular aspect, R₂ represents an acetyl group.

According to another particular aspect, R₃ is a hydrogen atom.

As a general rule, oil containing a high concentration of oleic acid will be chosen as a useful source of acylglycerols according to the invention. Such oil usually contains a high proportion of acylglycerols useful according to the invention.

According to a particular aspect of the invention, the preferred diglycerols of fatty acids are selected in the group consisting of 1,2-diolein and 1-oleoyl-2-acetyl glycerol.

A certain number of them, and more particularly those which are found to be the most active in the applications sought after, are also available commercially. This is the case particularly for 1-oleoyl-2-acetylglycerol and 1,2-dioleoylglycerol, which exist as commercial products with a high purity content. In particular, glycerol monooleate containing about 44 % of dioleic glycerol, from which about 14 % is 1,2-diolein. Such a compound is pharmaceutically accepted *(*European Pharmacopeia (4th Edition), *USP* 25/NF20, and *Japanese Standard of food Additives).* Such product is for instance commercially available by Gattefosse Company under the name PECEOL^{®}.

The acylglycerols are preferably incorporated or comprised in the composition or reverse-micelle system in an amount by weight ranging from 55 g to 90 g with respect to 100 g of the total weight of the composition or reverse-micelle system according to the invention.

### STEROLS

The sterols useful for the preparation of the reverse-micelle system according to the invention are preferably natural sterols, such as cholesterol or phytosterols (vegetable sterols). Sitosterol or cholesterol are the preferred sterols useful for the reverse-micelle system according to the invention.

Sitosterol and cholesterol are commercially available. More particularly, commercial sitosterol which is extracted from soya can be used. In such a product, the sitosterol generally represents from 50 to 70% by weight of the product and is generally found in a mixture with campesterol and sitostanol in respective proportions in the order of 15% each. Commercial sitosterol which is extracted from a variety of pine called tall oil can also be used. In general, it will be possible to use sitosterol in mixture with sitostanol. Preferably, said mixture comprises at least 50% sitosterol by weight of the mixture.

As mentioned above, the ratios of the lipidic constituents (sterols, acylglycerol and phospholipids) in the reverse-micelle system according to the invention can vary in a wide range, for instance the weight ratio sterols/acylglycerol can range from 0.015 to 0.05, more particularly from 0.03 to 0.04.

### PHOSPHOLIPIDS

Phospholipids are formed of a glycerol linked to 2 fatty acids and to a phosphate group. The variability of phospholipids relies on the fatty acids that are attached to the glycerol and on the chemical groups that are susceptible to link to the phosphate group. Phospholipids are the major lipidic constituents of biological membranes.

Among phospholipids useful in the present invention may be cited phosphatidylethanolamine, phosphatidylserine, phosphatidylglycerol, diphosphatidylglycerol, phosphatidylinositol, and phosphatidylcholine.

In a particular embodiment, the phospholipid is phosphatidylcholine. Phosphatidylcholine is also known as 1,2-diacyl-glycero-3-phosphocholine or PtdCho.

Phosphatidylcholine is formed from a choline, a phosphate group, a glycerol and two fatty acids. It is actually a group of molecules, wherein the fatty acid compositions vary from one molecule to another. Phosphatidylcholine may be obtained from commercial lecithin that contains phosphatidylcholine in concentrations of 20 to 98%. The lecithin preferably used for the preparation of the reverse micelles according to the invention is Epikuron 200^{®} and contains phosphatidylcholine at a concentration of more than 90%.

The weight ratio phospholipid/acylglycerol in compositions or reverse-micelle systems according to the invention is from 0.06 to 0.30.

### ALCOHOLS

The alcohols useful for the preparation of the reverse-micelle system according to the invention are preferably linear or branched mono-alcohols from C2 to C6. Examples of alcohols are ethanol, 1-butanol, 2-butanol, 3-methyl-1-butanol, 2-methyl-1-propanol, 1-pentanol, 1-propanol, 2-propanol and any mixture thereof. In a particular embodiment of the invention, alcohol is ethanol.

The alcohol is preferably incorporated or comprised in the composition or reverse-micelle system in an amount by weight ranging from 5 g to 17 g with respect to 100 g of the total weight of the composition or reverse-micelle system according to the invention.

### OLIGONUCLEOTIDES

The unmodified oligonucleotides targeting SARS-CoV-2 RNAs or targeting one or more genes of SARS-CoV-2 virus is a nucleic acid molecule capable of modulating gene expression by down regulating or knocking down the expression of a target nucleic acid sequence of SARS-CoV-2 virus.

Down regulating or knocking down the expression of a target nucleic acid sequence can be commonly accomplished via RNA interference (RNAi). RNAi generally designates a phenomenon by which dsRNA specifically reduces expression of a target gene at post-translational level. In normal conditions, RNA interference is initiated by double-stranded RNA molecules (dsRNA) of various length, for example ranging from 15 to 30 base pair length. *In vivo,* dsRNA introduced into a cell is cleaved into a mixture of short dsRNA molecules.

Nucleic acid molecules capable of modulating gene expression by down regulating or knocking down the expression of a target nucleic acid sequence SARS-CoV-2 virus can thus include "antisense oligonucleotides", "short interfering nucleic acid" (siNA), "short interfering RNA" (siRNA), "short interfering nucleic acid molecule", "short interfering oligonucleotide molecule", "miRNA", "micro RNA", guide RNA (gRNA), short guide RNA (sgRNA) of a CRISPR system, "short hairpin RNA" (shRNA) or a mixture thereof.

Unmodified oligonucleotides as defined above, such as unmodified siRNAs, are prone to rapid degradation by ubiquitous endo- and exonucleases and they are generally undetectable in the blood already 10 min after administration.

The oligonucleotides used in the present invention are necessarily chemically unmodified in order to be perfectly water-soluble. More specifically, unmodified oligonucleotides refer to oligonucleotides without any structural modifications at the ribose level (e.g. 2'-fluoro, 2'-methyl, and/or 2'-methoxy), at the base level and at the backbone level (e.g. phosphodiester, phosphorithioate).

According to a preferred embodiment, oligonucleotides of the present invention are at least 10, 15, 20 or 25 nucleotides (nt) long, more preferably in the range of 19 to 25 nucleotides long, or typically 19, 20, 21, 22, 23, 24 or 25 nt long.

The oligonucleotides of the present invention are designed to have complementarity to the target sequence. In the context of the present invention, they are more specifically designed to have complementarity to a target nucleic acid sequence of the SARS-CoV-2 virus genome.

The term RNA interference (RNAi) is used to describe gene silencing or knocking down at the mRNA level guided by small complementary non-coding RNA species. There are several classes of RNAi mediators, one of which, namely small interfering RNAs (siRNAs). The source of siRNAs during infection is viral double-stranded RNA (dsRNA), which is cleaved by cytoplasmic RNAse III family enzyme Dicer into 19-27 base pair (bp) long molecules with a perfectly complementary middle region and 2-nt overhangs on both 3' ends. These siRNAs are incorporated into a multiprotein RNA-induced silencing complex (RISC). Following the strand separation, the antisense strand (i.e. guide strand) guides the RISC to recognize and cut target RNA transcripts (the other strand is called passenger strand).

Unmodified oligonucleotides as defined above, such as unmodified siRNAs, are aimed at inhibiting or reducing contagiousness of SARS-CoV-2 virus, more specifically, by protecting host from viral infection, inhibiting the expression of viral antigen or accessory genes, controlling the transcription, retro transcription or replication of viral genome, hindering the assembly of viral particles, or displaying influences in virus-host interactions.

Unmodified oligonucleotides as defined above, such as unmodified siRNAs, can thus be used to protect host from viral infection, inhibit the expression of viral antigen and accessory genes, control the transcription, retro transcription or replication of viral genome, hinder the assembly of viral particles, or display influences in virus-host interactions.

Whether RNAi is a functional antiviral pathway in mammals is still contentious, since production of siRNA molecules from long dsRNAs cannot be explicitly demonstrated in mammalian cells due to the fact that dsRNA longer than 30 bp triggers activation of interferon (IFN) response which shuts down the natural RNAi. However, mammalian cells do possess all the components of evolutionary conserved RNAi machinery that can be harnessed to inhibit the expression of cognate mRNA by exogenous siRNA molecules. The antiviral potential of siRNAs was first demonstrated against respiratory syncytial virus and thereafter numerous studies describing antiviral activity of siRNAs against viruses with DNA and RNA genomes *in vitro* and *in vivo* have been published. RNAi-based drugs thus appear to be a viable option to treat severe viral infections, against which effective vaccines or specific cure is not available yet, such as Ebola virus or emerging viruses, in particular SARS-CoV-2 virus.

The first step in production of antiviral siRNAs is *in silico* selection of highly conservative sequences in the targeted virus genome in order to achieve strong antiviral activity and avoid off-target effects.

After internalization of siRNA duplexes in treated cells, the duplexes are loaded on proteins of the "Ago" family, forming a molecular complex named "RISC" (RNA-induced silencing complex). There are 4 Ago proteins in mouse and in human, but only one (called "Ago2") is able to degrade target RNAs by endonucleolytic cleavage. That reaction generally occurs when the guide strand and the target are highly complementary (a perfect match to the "seed" [preferably nucleotides 2-7 of the guide strand] is important for target binding; and a perfect match to the central part [preferably nucleotides 8-14] of the guide strand is important for target cleavage).

The oligonucleotides of the present invention are designed to have complementarity to a target nucleic acid sequence of SARS-CoV-2 virus genome. This complementarity involves at least 13 bases, typically between 13 and 25 bases, preferably at least 14 bases, even more preferably at least 18 bases of the oligonucleotides of the present invention.

The term "complementary" or "complementarity" refers herein to the ability of oligonucleotides to form base pairs with another nucleotide molecule. Base pairs are typically formed by hydrogen bonds between nucleotide units in antiparallel polynucleotide strands. Complementary polynucleotide strands can base pair in the Watson-Crick manner (e.g., A to T, A to U, C to G), or in any other manner that allows for the formation of duplexes. As persons skilled in the art are aware, when using RNA as opposed to DNA, uracil rather than thymine is the base that is considered to be complementary to adenosine. However, when a U is denoted in the context of the present invention, the ability to substitute a T is implied, unless otherwise stated. Perfect complementarity or 100 percent complementarity refers to the situation in which each nucleotide unit of the oligonucleotide strand of the invention can bind to a nucleotide unit of a second oligonucleotide strand. Less than perfect complementarity refers to the situation in which some, but not all, nucleotide units of two strands can bind with each other. For example, for two 20-mers, if only two base pairs on one strand of the invention (e.g. guide strand) can bind with the other, the oligonucleotide strand of the invention exhibits 10 percent complementarity. In the same way, if 20 base units of one 20 nt strand (e.g. guide strand) can be bond with 20 other base units of the target gene, the oligonucleotide strands of the invention exhibit 100 percent complementarity.

In a particular aspect, the oligonucleotides of the present invention is a double-stranded RNA (or RNA duplexes), in particular a small interfering RNA (siRNA), with a guide strand and a passenger strand.

The oligonucleotides of the present invention are synthetic RNA duplexes comprising or consisting of two unmodified 21-mer oligonucleotides annealed together to form short/small interfering RNAs (siRNAs).

The unmodified oligonucleotide targeting one or more genes of the virus SARS-CoV-2 advantageously targets constant regions of the virus genome.

More particularly the unmodified oligonucleotides target the Orflab gene of SARS-CoV-2.

The siRNA of the invention presents a guide strand which comprises, or consists of, one of the following sequences:
- 5' P-UAUAGCUAAAGACACGAACCGC 3';
- 5' P-UAAACUACAAAUGGAACACCAC 3';
- 5' P-UUGAGUGCAUCAUUAUCCAACC 3';
- 5' P-UUAGCUAAAGACACGAACCGUC 3' ;
- 5' P-UUUGAGUGCAUCAUUAUCCAC 3'.

Preferably, the siRNA of the invention presents a guide strand which comprises, or consists of, SEQ ID NO 1.

According to a preferred embodiment, the siRNA duplexes of the invention, with guide strand and passenger strand, comprises, or consists of, one of the following duplex sequences:
siRNA A
   - SEQ ID NO 1: guide strand: 5' P-UAUAGCUAAAGACACGAACCGC 3';
   - SEQ ID NO 2: passenger strand: 5' GGUUCGUGUCUUUAGUUAUUCU 3' siRNA B
   - SEQ ID NO 3: guide strand: 5' P-UAAACUACAAAUGGAACACCAC 3';
   - SEQ ID NO 4: passenger strand: 5' GGUGUUCUAUUUGUAGUUUUCU 3'
siRNA C
   - SEQ ID NO 5: guide strand: 5' P-UUGAGUGCAUCAUUAUCCAACC 3';
   - SEQ ID NO 6: passenger strand: 5' UUGGAUAAUGAUGCACUCUUCU 3'
siRNA D
   - SEQ ID NO 7: guide strand: 5' P-UUAGCUAAAGACACGAACCGUC 3' ;
   SEQ ID NO 8: passenger strand: 5' CGGUUCGUGUCUUUAGUUAUCU 3'
- siRNA E
   SEQ ID NO 9: guide strand: 5' P-UUUGAGUGCAUCAUUAUCCAC 3'.
   SEQ ID NO 10: passenger strand: 5'GGAUAAUGAUGCACUUAAUCU 3'

Preferably, the siRNA of the invention comprises, or consists of, the duplex sequences of SEQ ID NO 1 and 2 (siRNA "A").

The siRNA with guide stands corresponding to SEQ ID 1, 3, 5, 7 or 9, also comprise passenger strands as to form effective siRNAs duplexes, as described above.

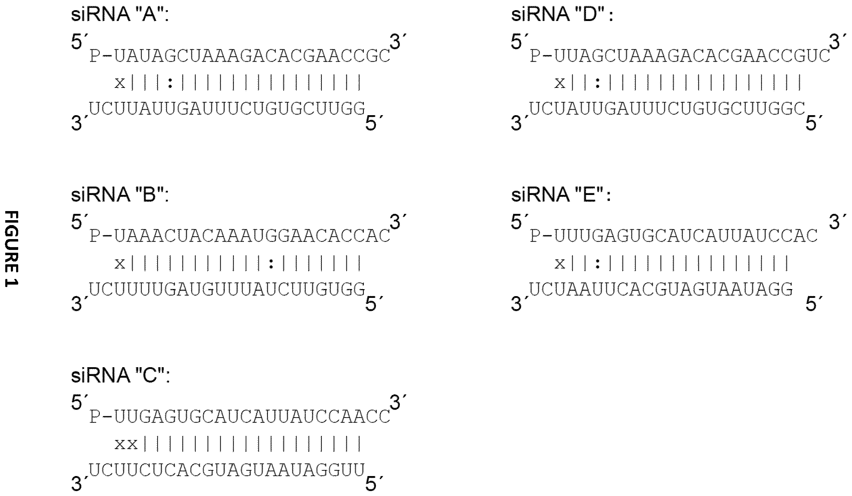

Schematic of said siRNA structures are dispatched in **FIGURE 1** (I: Watson-Crick base pair; x: mismatch; ·: GU wobble).

According to a particular aspect, the invention relates to the siRNAs as identified above.

According to another aspect, the invention relates a pharmaceutical composition comprising at least one the siRNAs as defined above, in a pharmaceutically acceptable carrier or excipient.

### USE OF REVERSE MICELLES TO DELIVER UNMODIFIED NUCLEOTIDES TARGETING VIRUS SARS-CoV-2 GENES

The pharmaceutical composition of the present invention describes unmodified oligonucleotides, such as siRNAs, targeting one or more genes of the SARS-CoV-2 Coronavirus virus, formulated in the microemulsion (or reverse micelles) as described herein and intended for the treatment of patients contaminated by this virus.

The unmodified oligonucleotides, such as siRNAs, targeting one or more genes of the SARS-CoV-2 are present in the aqueous core of the reverse micelles.

The amount of unmodified oligonucleotides, such as siRNAs, targeting one or more genes of SARS-CoV-2 incorporated into the reverse micelle system is determined by their solubility in the hydrophilic phase (aqueous core). Preferably, the amount of unmodified oligonucleotides, such as siRNAs, targeting one or more genes of SARS-CoV-2 included in the reverse micelle system depends on their size.

The reverse micelles of the invention allow the oligonucleotide included therein to be administered and transported to cells with a high degree of protection in lipoprotein HDL and vHDL, in particular without affecting its stability.

It is known today that a reverse-micelle system can be used for the preparation of nanomaterials, which act as micro reactors. The activity and stability of bio molecules can be controlled, mainly by the concentration of water in this medium.

An object of the invention concerns a pharmaceutical composition comprising reverse micelles as defined above and at least a pharmaceutically acceptable carrier, excipient or support, more specifically for use in the treatment of COVID-19.

According to a particular embodiment, the pharmaceutical composition is in the form of airless bottle, a capsule, a caplet, an aerosol, a spray, a solution or a soft elastic gelatin capsule.

A further object of the invention concerns the use of reverse micelles as defined above for preparing a pharmaceutical composition intended for the treatment of COVID-19.

The present invention further concerns a method for the treatment of COVID-19, wherein the method comprises the step of administering into a subject in need of such treatment a therapeutically efficient amount of one or more unmodified oligonucleotides as defined above.

More specifically, administration of one or more unmodified oligonucleotides in reverse micelle system as defined herein or pharmaceutical composition comprising the same is a mucosal delivery.

As pharmaceutically acceptable excipient, vehicle or carrier, any excipient, vehicle or carrier well-known to the person skilled in the art may be used. Other additives well-known to the person skilled in the art such as stabilisers, drying agents, binders or pH buffers may also be used. Preferred excipients in accordance with the invention promote adherence of the finished product to the mucosa.

The compositions of the invention can be administered in different ways, in particular via the oral, nasal, vaginal or rectal route, with a buccal, nasal, vaginal or digestive absorption, or more generally via mucosal tissue absorption. The composition of the invention is preferably administered by buccal route or rectal route, with a buccal mucosa or rectal mucosa absorption, respectively.

Within the context of the invention, the term treatment denotes curative, symptomatic, and preventive treatment. As used herein, the term "treatment" of COVID-19 refers to any act intended to extend life span of subjects (or patients) such as therapy and retardation of the disease progression. The treatment can be designed to eradicate the disease, to stop the progression of the disease, and/or to promote the regression of the disease. The term "treatment" of a disease also refers to any act intended to decrease one or more mild symptoms associated with the disease, including fever, cough, shortness of breath, muscle pain, sputum production and/or sore throat. The term "treatment" of the disease also refers to any act intended to decrease one or more severe symptoms associated with the disease, including lung damage, pneumonia and/or multi-organ failure. More specifically, the treatment according to the invention is intended to delay the appearance of, alleviate, or hinder, the mild symptoms and more particularly the severe symptoms of COVID-19, such as COVID-19 associated lung damage, pneumonia or multi-organ failure.

In particular embodiments, the SARS-CoV-2 includes one or several of SARS-CoV-2 lineages or SARS-CoV-2 variants such as :
Alpha variant (B.1.1.7+Q.*)
Beta variant (B.1.351+B.1.351.2+B.1.351.3)
Gamma variant (P.1+P.1.*)
Delta variant (B.1.617.2+AY.*)
Eta variant (B. 1.525)
Iota variant (B. 1.526)
Kappa variant (B.1.617.1)
Lambda variant (C.37+C.37.1)
Mu variant (B.1.621+B.1.621.1)

As used herein, the term "therapeutically effective amount" is intended an amount of unmodified oligonucleotides as defined above, administered to a patient that is sufficient to constitute a treatment of COVID-19 as defined above. In a particular embodiment, the therapeutically effective amount to be administered is an amount sufficient to down regulate or knock down the expression of a target nucleic acid of SARS-CoV-2 virus. The amount of unmodified oligonucleotides as defined above to be administered can be determined by standard procedure well known by those of ordinary skill in the art. Physiological data of the patient (e.g. age, size, and weight), the routes of administration and the disease to be treated have to be taken into account to determine the appropriate dosage. One skilled in the art will recognize that the amount of unmodified oligonucleotides to be administered will be an amount that is sufficient to induce reduction of COVID-19 symptoms or to induce alleviation of one or more symptoms of COVID-19.

The subject (or patient) to treat is any mammal, preferably a human being. Preferably, the subject is a human patient, whatever its age or sex. New-borns, infants, children are included as well. More preferably, the patient or subject according to the invention is suspected to be infected by SARS-CoV-2 or has been diagnosed to have CoVID-19 or has been diagnosed as infected by SARS-CoV-2. The standard method of diagnosis is by reverse transcription polymerase chain reaction (rRT-PCR) from a nasopharyngeal swab or throat swab. The infection can also be diagnosed from a combination of symptoms, risk factors and a chest CT scan (Computer Tomography scan) showing features of pneumonia.

As used herein, the terms "mucosa" and "mucosal" refer to a mucous tissue such as of the respiratory, digestive, or genital tissue. "Mucosal delivery", "mucosal administration" and analogous terms as used herein refer to the administration of a composition through a mucosal tissue. "Mucosal delivery", "mucosal administration" and analogous terms include, but are not limited to, the delivery of a composition through preferably buccal administration, bronchi, gingival, lingual, nasal, buccal, vaginal, rectal, and gastro-intestinal mucosal tissue. Administration according to the invention is more preferably carried out via buccal mucosa or rectal mucosa.

### EXAMPLES

The following examples are intended to exemplify the operation of the present invention but not to limit its scope.

### Example 1: Ex vivo assessment of the anti-viral activity

### 2.1. Cell viability

VeroE6 cells were transfected with varying concentrations (InM, 5nM, 20nM and 100nM) of siRNAs A to E. After transfection the cells were infected with the EBH SARS-CoV-2 strain (isolated at the Montpellier University Hospital). The viability of the cells was measured 72 hours later (measured by the abundance of ATP in the cell lysate of the culture). Six biological replicates were made for each experimental condition.

Additional controls were run, with non-transfected cells, infected or not, and treated or not with 10 µM of remdesivir.

In an independent experiment, the cells were treated under the same conditions, then 48 hours after infection, the RNAs of the cells were extracted, and the viral RNA was quantified by RT-qPCR (by amplification of the RNA of the viral gene E, and normalized by the abundance of cellular GAPDH mRNA).

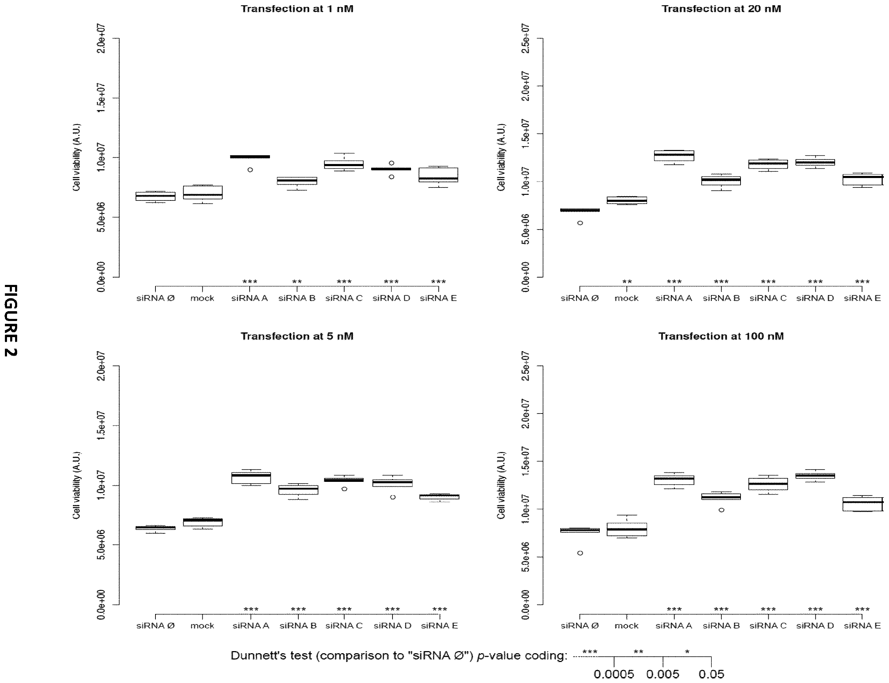

### As shown on Figure 2, transfection of various concentrations of siRNAs in VeroE6 cells significantly improved cell viability after SARS-CoV-2 infection.

### 2.1. Measurement of viral RNA

After transfection of 20 nM of each siRNA A to E, the VeroE6 cells were infected with the EBH SARS-CoV-2 strain (isolated at the Montpellier University Hospital), then their RNA (viral and cellular GAPDH) were quantified for 48 hours later (assessed by RT-qPCR). Six biological replicates were made for each experimental condition.

Additional controls were run, with non-transfected cells, infected or not, and treated or not with 10 µM of remdesivir.

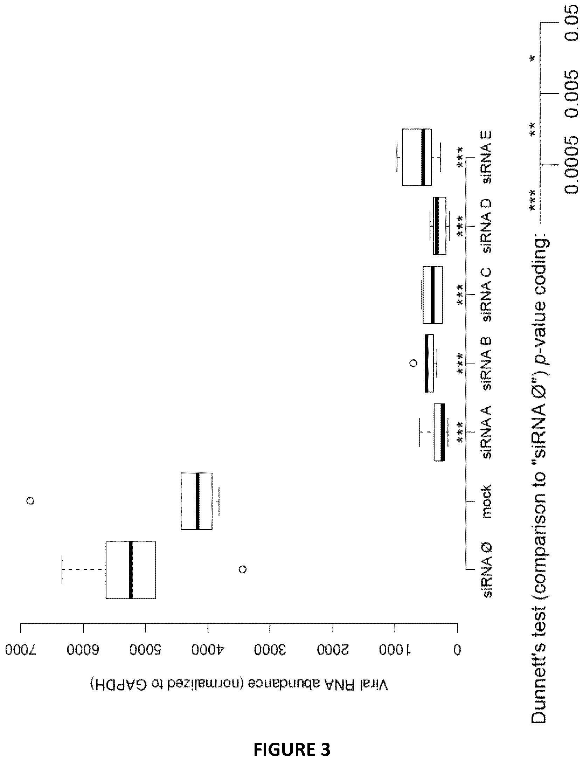

### As shown on Figure 3, transfection of 20 nM siRNA in VeroE6 cells significantly reduced viral replication.

### Example 2: Production of the reverse microemulsion

The aim of this study was to evaluate by visual determination the formulation and the stability of the siRNAs A to E targeting SARS-CoV-2 in the reverse microemulsion.

8.5 g of lecithin were dissolved in 6.8 g of absolute ethanol by magnetic stirring at 300 r/min for 15 minutes at room temperature. 1.4 g of sitosterol were added to the mixture and stirred in the same conditions. 32.6 g of glycerol monooleate was added thereto and magnetic stirring was carried out at 500 r/min for 45 minutes at 37°C.

168 mg of a siRNA aqueous solution containing 1,06 mg of siRNA were added to 1148.0 mg of the oil mixture as prepared above and then stirred at room temperature by magnetic stirring at 700 r/min for 30 minutes.

## Claims

1. Reverse micelle system comprising at least one sterol, acylglycerol, phospholipid, an alcohol, water and an unmodified oligonucleotide targeting a gene of SARS-CoV-2 virus, wherein the unmodified oligonucleotide is a synthetic RNA duplex comprising or consisting of two unmodified oligonucleotides annealed together to form an siRNA, wherein the siRNA presents a guide strand which comprises, or consists of, one of the following sequences:
- 5' P-UAUAGCUAAAGACACGAACCGC 3' (SEQ ID NO : 1);
- 5' P-UAAACUACAAAUGGAACACCAC 3' (SEQ ID NO : 3);
- 5' P-UUGAGUGCAUCAUUAUCCAACC 3' (SEQ ID NO : 5);
- 5' P-UUAGCUAAAGACACGAACCGUC 3' (SEQ ID NO : 7);
- 5' P-UUUGAGUGCAUCAUUAUCCAC 3' (SEQ ID NO : 9).

2. The reverse micelle system according to claim 1, wherein the micelles present aqueous cores of around 4 nm, preferably from 3 to 5 nm, more preferably from 3.5 to 5 nm, in particular from 3.7 to 4.5 nm.

3. The reverse micelle system according to claim 1 or 2, wherein acylglycerol presents the following formula (I): 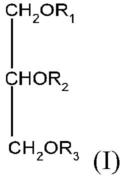 in which:
- R₁ is an acyl residue of a linear or branched, saturated or unsaturated fatty acid having between 14 and 24 carbon atoms, a hydrogen atom, or a mono-, di- or tri-galactose or glucose;
- R₂ is an acyl residue of a linear or branched, saturated or unsaturated fatty acid having between 2 and 18 carbon atoms;
- R₃ is an acyl residue of a linear or branched, saturated or unsaturated fatty acid having between 14 and 24 carbon atoms, or a hydrogen atom.

4. The reverse micelle system according to any one of claims 1-3, wherein the at least one sterol is sitosterol, and/or phospholipid is lecithin, and/or alcohol is ethanol, and/or acylglycerol is glycerol monooleate.

5. A pharmaceutical composition comprising a reverse micelle system as defined in any one of claims 1-4, and at least a pharmaceutically acceptable carrier.

6. A method for the preparation of the reverse micelle system as defined in any one of the preceding claims, wherein it comprises the following steps
(a) Contacting (i) sterol, (ii) acylglycerol, preferably diacylglycerol of fatty acids, (iii) phospholipid, preferably phosphatidylcholine, (iv) alcohol, (v) water, preferably purified water, and (vi) the siRNA as defined in claim 1,
(b) Stirring the mixture obtained in step (a), at 40 °C or less, and for a time sufficient to obtain formation of reverse micelles, said stirring being carried out mechanically.

7. A siRNA presenting a guide strand which comprises, or consists of, one of the following sequences:
- 5' P-UAUAGCUAAAGACACGAACCGC 3' (SEQ ID NO : 1);
- 5' P-UAAACUACAAAUGGAACACCAC 3' (SEQ ID NO :3);
- 5' P-UUGAGUGCAUCAUUAUCCAACC 3'(SEQ ID NO :5);
- 5' P-UUAGCUAAAGACACGAACCGUC 3'(SEQ ID NO :7) ;
- 5' P-UUUGAGUGCAUCAUUAUCCAC 3' (SEQ ID NO :9).

8. A siRNA duplex, with guide strand and passenger strand, which comprises, or consists of, one of the following duplex sequences:
siRNA A
- SEQ ID NO 1: guide strand: 5' P-UAUAGCUAAAGACACGAACCGC 3';
- SEQ ID NO 2: passenger strand: 5' GGUUCGUGUCUUUAGUUAUUCU 3' siRNA B
- SEQ ID NO 3: guide strand: 5' P-UAAACUACAAAUGGAACACCAC 3';
- SEQ ID NO 4: passenger strand: 5' GGUGUUCUAUUUGUAGUUUUCU 3' siRNA C
- SEQ ID NO 5: guide strand: 5' P-UUGAGUGCAUCAUUAUCCAACC 3';
- SEQ ID NO 6: passenger strand: 5' UUGGAUAAUGAUGCACUCUUCU 3' siRNA D
- SEQ ID NO 7: guide strand: 5' P-UUAGCUAAAGACACGAACCGUC 3' ;
- SEQ ID NO 8: passenger strand: 5' CGGUUCGUGUCUUUAGUUAUCU 3' siRNA E
- SEQ ID NO 9: guide strand: 5' P-UUUGAGUGCAUCAUUAUCCAC 3'.
- SEQ ID NO 10: passenger strand: 5'GGAUAAUGAUGCACUUAAUCU 3'.

9. A pharmaceutical composition comprising at least one the siRNAs or siRNA duplexes as defined in any one of claims 7 or 8, in a pharmaceutically acceptable carrier.

10. The composition according to claim 5 or 9, for use in treating COVID-19.

11. The composition according to claim 5 or 9, for use in treating COVID-19 associated lung damage or multi-organ failure.

12. The composition for use according to claims 10 or 11, wherein the composition is administered by oral, buccal or rectal route.
